# FASCICULE DE BREVET EUROPEEN

(11) **EP 1 466 589 B1**
(45) Date de publication et mention de la délivrance du brevet: **17.12.2008**
(21) Numéro de dépôt: 04290646.1
(22) Date de dépôt: 10.03.2004
(51) Int. Cl.: A61K 8/06, A61K 8/34, A61K 8/39, A61K 8/894, A61Q 19/00, A61Q 19/06

(54) **Composition cosmétique stable contenant un glycéride d'acides gras, un alcool et un émulsionnant siliconé particulier**
Stabile kosmetische Zusammensetzung enthaltend ein Fettsäureglycerid, einen Alkohol und einen spezifischen Silikonemulgator
Stable cosmetic composition comprising a fatty acid glyceride, an alcohol and a silicone emulsifier

(30) Priorité: 09.04.2003 FR 0304399
(43) Date de publication de la demande: 13.10.2004
(73) Titulaire: L'ORÉAL, 75008 Paris (FR)
(72) Inventeur: Meunier, Christine, 92350 Le Plessis Robinson (FR)
(74) Mandataire: Kromer, Christophe

(56) Documents cités:
- US-A- 6 007 799
- US-B1- 6 383 503
- DATABASE WPI Section Ch, Week 199912 Derwent Publications Ltd., London, GB; Class A26, AN 1999-136679 XP002265284 -& JP 11 005712 A (SHISEIDO CO LTD) 12 janvier 1999 (1999-01-12)

## Description

L'invention se rapporte à une composition se présentant sous forme d'une émulsion eau-dans-huile (E/H), contenant un glycéride d'acide gras polyoxyéthyléné et/ou polyoxypropyléné, un mono-alcool et un tensioactif siliconé particulier. Cette composition est utilisable en particulier dans les domaines cosmétique et/ou dermatologique.

Il est courant d'utiliser des compositions cosmétiques ou dermatologiques constituées d'une émulsion eau-dans-huile (E/H) comportant une phase aqueuse dispersée dans une phase huileuse. Ces émulsions comportent une phase continue huileuse et permettent donc de former à la surface de la peau un film lipidique, ce qui a pour conséquence de prévenir la perte d'eau transépidermique et de protéger la peau des agressions extérieures. Ces émulsions sont donc particulièrement appropriées pour protéger et nourrir la peau, mais également pour toutes les applications classiques de la cosmétique, et qui dépendront des actifs contenus dans les compositions.

Toutefois, les crèmes sous forme d'émulsions E/H présentent l'inconvénient d'apporter sur la peau à l'application, un toucher assez gras, la phase huileuse étant la phase externe. Ainsi, ces crèmes sont en général utilisées pour les peaux sèches, étant trop grasses pour être utilisées sur les peaux grasses. De plus, les émulsions E/H n'apportent aucune fraîcheur et sont généralement trop riches en huiles pour être utilisées pendant l'été ou dans les pays chauds.

Pour surmonter ces inconvénients, il a été envisagé de préparer des émulsions à forte teneur en alcool. Ces émulsions, outre le gain en fraîcheur, présentent des avantages supplémentaires comme par exemple une meilleure pénétration dans la peau, et un aspect beaucoup plus gélifié et transparent.

Toutefois, la teneur en alcool ne peut pas être trop importante pour des raisons de tolérance qui peuvent entraîner des problèmes d'irritations cutanées notamment chez les sujets à peaux sensibles. De plus la présence d'une quantité importante d'alcool peut engendrer des problèmes supplémentaires en terme de stabilité ou d'incompatibilité avec d'autres matières premières couramment utilisées. C'est le cas en particulier avec certains tensioactifs ou émollients tels que les glycérides d'acides gras, ou leurs dérivés. Ces derniers sont utilisés soit pour leurs caractéristiques propres, soit parce qu'ils sont présents dans de nombreuses matières premières commerciales en tant que solvant. Il devient alors difficile de préparer des émulsions E/H stables contenant ces matières premières. En présence d'une quantité importante d'éthanol, les émulsions ainsi obtenues présentent une stabilité dans le temps très médiocre, et ont même parfois tendance à déphaser immédiatement après leur fabrication.

La Demanderesse a mis en évidence de manière fortuite que l'utilisation d'un émulsionnant siliconé particulier permettait de palier ces inconvénients dus à une quantité importante de mono-alcool en présence de certains tensioactifs ou émollients, et d'obtenir des compositions sous forme d'émulsions eau-dans-huile stables dans le temps.

La présente invention a donc pour objet une composition cosmétique comprenant :
(a) au moins une phase aqueuse dispersée dans une phase grasse,
(b) au moins un glycéride d'acide gras ou de mélange d'acides gras en C₆ à C₂₂ polyoxyéthyléné et/ou polyoxypropyléné,
(c) de 10 à 50 % en poids, par rapport au poids total de la composition, d'au moins un mono-alcool en C₂-C₃,
(d) au moins un émulsionnant siliconé de formule (I) : 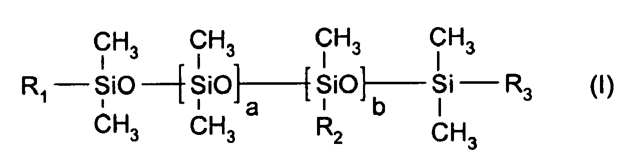 dans laquelle :
   a est un nombre entier allant de 0 à 400,
   b est un nombre entier allant de 0 à 50,
   a et b ne pouvant être simultanément égaux à 0,
   R₁, R₂, R₃, représentent indépendamment un radical alkyle en C₁-C₆, ou un radical -(CH₂)ₓ-(OCH₂CH₂)_{y}-(OCH₂CH₂CH₂)_{z}-OR₄, au moins un des radicaux R₁, R₂, R₃ étant différent d'un radical alkyle,
   R₄ représente un atome d'hydrogène, un radical alkyle en C₁-C₃, ou un radical acyle en C₂-C₄,
   x est un nombre entier allant de 0 à 6,
   y est un nombre entier allant de 1 à 30,
   z est un nombre entier allant de 0 à 30.

Selon un mode de réalisation préféré de l'invention, dans le composé de formule (I), R₁ et R₃ représentent chacun un radical méthyle, et R₂ un radical -(CH₂)ₓ-(OCH₂CH₂)_{y}-(OCH₂CH₂CH₂)₂-OR₄ dans lequel a est un entier allant de 300 à 400, b est un entier allant de 1 à 10, et y et z sont des entiers allant de 10 à 20 choisis indépendamment. Dans ces composés, R₄ sera préférentiellement un atome d'hydrogène, et x un entier allant de 2 à 4.

Comme composé de formule (I) convenant à la mise en oeuvre de la présente invention, on peut citer les diméthicones copolyols vendus sous les dénominations DC 5329, DC 7439-146, DC 2-5695 par la société Dow Corning ; KF-6013, KF-6015, KF-6016, KF-6017 par la société Shin-Etsu.

Les composés DC 5329, DC 7439-146, DC 2-5695 sont des composés de formule (I) où R₁ et R₃ représentent chacun un groupement CH₃, R₂ représente un radical -(CH₂)ₓ-(OCH₂CH₂)_{y}-(OCH₂CH₂CH₂)_{z}-OR₄ où x est 2, z est 0, R₄ est H, et respectivement a est 22, b est 2 et y est 12 ; a est 103, b est 10 et y est 12 ; a est 27, b est 3 et y est 12.

Plus particulièrement, pour la mise en oeuvre de la présente invention, on préférera un composé de formule (II) : 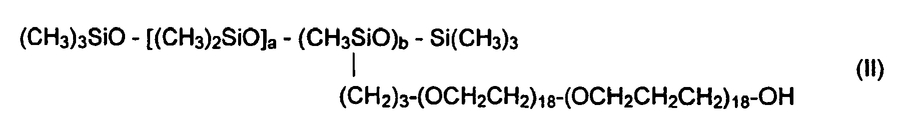 dans laquelle a est un entier allant de 300 à 400, et b est un entier allant de 1 à 10, et de façon préférentielle, a vaut 394 et b vaut 4.

L'émulsionnant siliconé peut être présent dans la composition selon l'invention en une quantité comprise entre 0,1 % et 5% en poids par rapport au poids total de la composition, préférentiellement en une quantité comprise entre 0,5 et 3 % en poids.

Selon un autre mode de réalisation, l'émulsionnant siliconé de formule (I) est associé à une cyclométhicone de formule (III) : 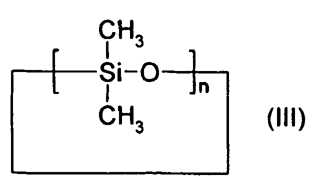 dans laquelle n est un nombre entier compris entre 3 et 8.

On peut citer en particulier le cyclotétrasiloxane (n=4), le cyclopentasiloxane (n=5), et le cyclohexasiloxane (n=6). Le cyclopentasiloxane est une silicone particulièrement adaptée à la mise en oeuvre de la présente invention.

La cyclométhicone peut être présente en une quantité allant de 5 à 40 % en poids par rapport au poids total de la composition, préférentiellement de 10 à 30 % en poids.

Le rapport entre l'émulsionnant siliconé de formule (I) et la cyclométhicone varie entre 0,0025 et 1, plus particulièrement entre 0,016 et 0,3.

Parmi les associations émulsionnant siliconé / cyclométhicone, on peut citer :
- Un mélange de composé de formule (II), de cyclotétrasiloxane et d'eau (rapport pondéral 10/88/2), commercialisé sous la dénomination DC 3225C par la société Dow Corning ;
- Un mélange de composé de formule (II), de cyclopentasiloxane et d'eau (rapport pondéral 10/88/2), commercialisé sous la dénomination DC2 5225C par la société Dow Corning;
- Un mélange de composé de formule (II) et de cyclopentasiloxane (rapport pondéral 43/57), commercialisé sous la dénomination DC 5185C par la société Dow Corning.

Selon un mode de réalisation particulier de l'invention, le mélange émulsionnant siliconé de formule (I) / cyclométhicone est l'unique système émulsionnant de la formule.

Dans le cadre de la présente invention, on désigne par glycéride d'acide gras un mélange de mono-, di-, et triester de glycérine et d'acide gras, et par glycéride de mélange d'acide gras, un mélange de mono-, di-, et triester de glycérine et d'un mélange d'acides gras.

Les glycérides d'acide gras ou de mélange d'acides gras polyoxyéthylénés et/ou polyoxypropylénés au sens de la présente invention se présentent sous la forme d'un dérivé de poly(éthylène)glycol et/ou de poly(propylène)glycol et de mélange de mono-, di-, et triglycéride d'acide gras ou de mélanges d'acides gras.

Dans un aspect préféré de l'invention, les glycérides d'acide gras polyoxyéthylénés et/ou polyoxypropylénés sont des glycérides de mélange d'acide gras, et plus particulièrement de mélange d'acides caprylique et caprique, polyoxyéthylénés et/ou polyoxypropylénés.. On choisira notamment les dérivés de polyéthylèneglycol (4OE ou 6OE) et de mélange de mono-, di-, et triglycéride d'acides caprylique et caprique. On peut citer en particulier le Glycérox 767 commercialisé par la société Croda.

La quantité de glycéride d'acide gras ou de mélange d'acides gras polyoxyéthyléné et/ou polyoxypropyléné dans la composition selon l'invention peut varier entre 0,001 % et 20 %, préférentiellement entre 0,1 % et 10 %, et plus particulièrement entre 0,1 % et 5 % en poids par rapport au poids total de la composition.

Le mono-alcool présent dans les compositions selon l'invention contient 2 ou 3 atomes de carbone en chaîne linéaire ou ramifiée, et peut donc être choisi parmi l'éthanol, le propanol et l'isopropanol. De préférence on choisira l'éthanol. Le mono-alcool est présent dans une quantité allant de 10 à 50 % en poids par rapport au poids total de la composition, préférentiellement dans une quantité comprise entre 10 % et 40 % en poids.

De préférence, la composition selon l'invention est destinée à un soin ou un traitement topique. Dans ce cas, l'émulsion doit contenir un milieu physiologiquement acceptable, c'est-à-dire compatible avec la peau, les muqueuses, les ongles, le cuir chevelu et/ou les cheveux. En outre, elle contient de préférence au moins un actif et trouve son application dans un grand nombre de traitements cosmétiques et/ou dermatologiques de la peau, y compris du cuir chevelu, des cheveux, des ongles, et/ou des muqueuses, en particulier pour le soin et/ou le maquillage et/ou la protection solaire de la peau et/ou des muqueuses, ainsi que pour la préparation d'une crème destinée au traitement des maladies de la peau.

Selon un mode de réalisation préféré, la composition selon l'invention contient au moins un actif lipolytique ou ayant une activité favorable, directe ou indirecte, sur la diminution du tissu adipeux. Parmi ces dérivés on peut trouver les inhibiteurs de phosphodiestérase, les extraits végétaux et les extraits d'origine marine, qui sont soit actifs sur les récepteurs à inhiber, tels que les β-2-bloqueurs, les NPY-bloqueurs (décrits dans le brevet EP-0 838 217), soit inhibent la synthèse des récepteurs aux LDL ou VLDL, soit actifs pour stimuler les récepteurs β et les protéines G, conduisant à l'activation de l'adénylcyclase, les peptides dérivés de l'hormone parathyroidienne tels que décrits dans les brevets FR-2 788 058 et FR-2 781 231 de Séderma ou les peptides décrits dans le document FR 2 786 693 voire tout autre peptide ayant des propriétés lipolytiques, les protamines et leurs dérivés tels que ceux décrits dans le document FR-2 758 724.

A titre d'exemple on peut citer les dérivés xanthiques comme la caféine et ses dérivés, notamment les 1-hydroxyalkylxanthines décrites dans le document FR-2 617 401, le citrate de caféine, la théophylline et ses dérivés, la théobromine, l'acéfylline, l'aminophylline, la chloroéthylthéophylline, le diprofylline, le diniprophylline, l'étamiphylline et ses dérivés, l'étofylline, la proxyphylline, les associations contenant des dérivés xanthiques, comme l'association de caféine et de silanol (dérivé méthylsilanetriol de caféine), et par exemple le produit commercialisé par la société Exsymol sous la dénomination caféisilane C, les composés d'origine naturelle contenant des bases xanthiques et notamment de la caféine, tels que les extraits de thé, de café, de guarana, de maté, de cola (*Cola Nitida*) et notamment l'extrait sec de fruit de guarana (*Paulina sorbilis*) contenant 8 à 10 % de caféine ; l'éphédrine et ses dérivés qui peuvent notamment se retrouver à l'état naturel dans les plantes telles que le Ma Huang (Ephedra plant), les extraits végétaux de *Garcinia Cambogia*, les extraits de *Bupleurum chinensis*, les extraits de lierre grimpant (*Hedera Helix*), d'arnica (*Arnica Montana L*), de romarin (*Rosmarinus officinalis N*), de souci *(Calendula officinalis),* de sauge (*Salvia officinalis L*), de ginseng *(Panax ginseng),* de millepertuis (*Byperycum Perforatum*), de fragon *(Ruscus aculeatus L),* d'ulmaire (*Filipendula ulmaria L*), d'orthosiphon (*Orthosiphon Stamincus Benth*), de bouleau *(Betula alba),* de cécropia et d'arganier, les extraits de ginkgo biloba, les extraits de prêle, les extraits d'escine, les complexes de phospholipide et de proanthocyanidines d'écorce de marron d'inde, les extraits de cangzhu, les extraits de *chrysanthellum indicum*, les sapogénines telles que la diosgénine, l'hécogénine, leurs dérivés et les extraits naturels en contenant, en particulier le Wild Yam, les extraits des plantes du genre *Armeniacea, Atractylodis Platicodon, Sinom-menum, Pharbitidis, Flemingia*, les extraits de *Coleus* tels que *C. Forskohlii, C. blumei, C. esquirolii, C. scutellaroïdes , C. xanthantus* et *C. Barbatus*, tel que l'extrait de racine de *Coleus Barbatus* contenant 60 % de forskoline, les extraits de Ballote, les extraits de *Guioa,* de *Davallia*, de *Terminalia*, de *Barringtonia*, de *Trema, d'Antirobia*, les extraits d'algues ou de phytoplancton, tels que le rhodystérol ou l'extrait de *Laminaria Digitata* commercialisé sous la dénomination PHYCOX75 par la société Secma, l'algue skeletonema décrite dans le brevet FR-2 782 921 ou les diatomées décrites dans le brevet FR-2 774 292.

La quantité d'actif(s) lipolytique(s) peut varier dans une large mesure et dépend de la nature de ou des actifs utilisés. De manière générale, le ou les actifs amincissants sont présents en une concentration allant de 0,001 à 20 % et de préférence de 0,1 à 10 % en poids par rapport au poids total de la composition.

Dans un mode de réalisation particulier, la composition selon l'invention contient au moins un extrait de Dioscorée riche en diosgénine, par exemple provenant de racines d'igname sauvage. On pourra par exemple choisir un extrait de racines de Dioscorea opposita commercialisé en solution dans un dérivé de polyéthylèneglycol (6OE) et de mélange de mono-, di-, et triglycéride d'acides caprylique et caprique / conservateurs / glycérine (rapport pondéral 1/93,8/0,2/5), commercialisé sous la dénomination Dioschol par la société Sederma.

La composition selon l'invention peut également contenir au moins un autre actif classiquement utilisé en cosmétique, tels que les agents desquamants capables d'agir, soit en favorisant l'exfoliation, soit sur les enzymes impliquées dans la desquamation ou la dégradation des cornéodesmosomes, les agents hydratants, les agents dépigmentants ou pro-pigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les agents agissant sur la microcirculation, et les agents agissant sur le métabolisme énergétique des cellules.

L'actif peut être par exemple présent en une concentration allant de 0,01 à 20 %, de préférence de 0,1 à 5 % et mieux de 0,5 à 3 % du poids total de la composition.

De façon connue, la composition de l'invention peut contenir également des adjuvants habituels dans les domaines cosmétique et/ou dermatologique, tels que les conservateurs, les antioxydants, les agents complexants, les solvants, les parfums, les charges, les filtres, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, ou dans la phase aqueuse.

La phase huileuse de la composition selon l'invention peut renfermer, outre l'huile de silicone éventuellement en mélange avec l'agent émulsionnant, toute sorte d'huiles et de corps gras bien connus de l'homme du métier, comme les huiles d'origine végétale telle que l'huile de noyau d'abricot, les huiles d'origine animale, les huiles hydrocarbonées telles que l'isohexadécane, l'isododécane, les isoparafines, les huiles de silicone, les huiles fluorées et/ou les huiles minérales, les huiles essentielles, les parfums, ainsi que les mélanges de ces huiles.

La phase huileuse peut contenir, en outre, d'autres constituants gras tels que les alcools gras et les acides gras.

La phase huileuse est présente dans la composition selon l'invention en une quantité allant de 5 à 40 %, de préférence de 8 à 22 % et de plus particulièrement de 12 à 20 % en poids par rapport au poids total de la composition.

Un autre avantage de la composition selon l'invention provient de ce qu'on peut y incorporer une grande quantité d'électrolyte sans nuire à la stabilité de la composition.

Comme électrolyte, on peut citer par exemple les sels des métaux mono-, di- ou trivalents, et plus particulièrement les sels de métal alcalino-terreux tels que les sels de baryum, de calcium et de strontium ; les sels de métal alcalin tels que les sels de sodium et de potassium, les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les bromures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate).

De préférence, l'électrolyte est un sel choisi parmi les sels de calcium, de magnésium, et de sodium, et notamment les chlorures et les sulfates, en particulier le sulfate de magnésium, ou un mélange comprenant au moins du chlorure de magnésium, du chlorure de potassium, du chlorure de sodium, du chlorure de calcium, du bromure de magnésium, le dit mélange correspondant à des sels de la mer morte.

La teneur en électrolyte, lorsqu'il est présent, va en général de 0,1 à 5 % et de préférence de 1 à 5 % en poids par rapport au poids total de la composition.

La présente invention a encore pour objet un procédé de traitement cosmétique de la peau, des cheveux, des ongles, du cuir chevelu et/ou des muqueuses, caractérisé par le fait que l'on applique sur la peau, les cheveux, les ongles, le cuir chevelu et/ou les muqueuses, une composition telle que définie ci-dessus.

Les exemples qui suivent illustrent l'invention sans en limiter la portée. Les composés sont, selon le cas, cités en noms chimiques ou en noms CTFA (International Cosmetic Ingredient Dictionary and Handbook).

### Exemple A: Mise en évidence de la stabilisation des compositions selon l'invention :

Le but de cet exemple est de mettre en évidence la stabilité des compositions selon l'invention contenant l'émulsionnant siliconé de formule (I).

Les émulsions des compositions 1 à 4 ont été préparées de manière classique pour l'homme du métier. Les compositions ainsi obtenues ont été observées afin d'évaluer leur stabilité à T=0, puis après 24, 48 heures, 2 mois à température ambiante, ainsi qu'après 2 mois à 4°C et à 45°C.

Les résultats ont été regroupés dans le tableau 1 (les quantités sont exprimées en pourcentage en poids) :

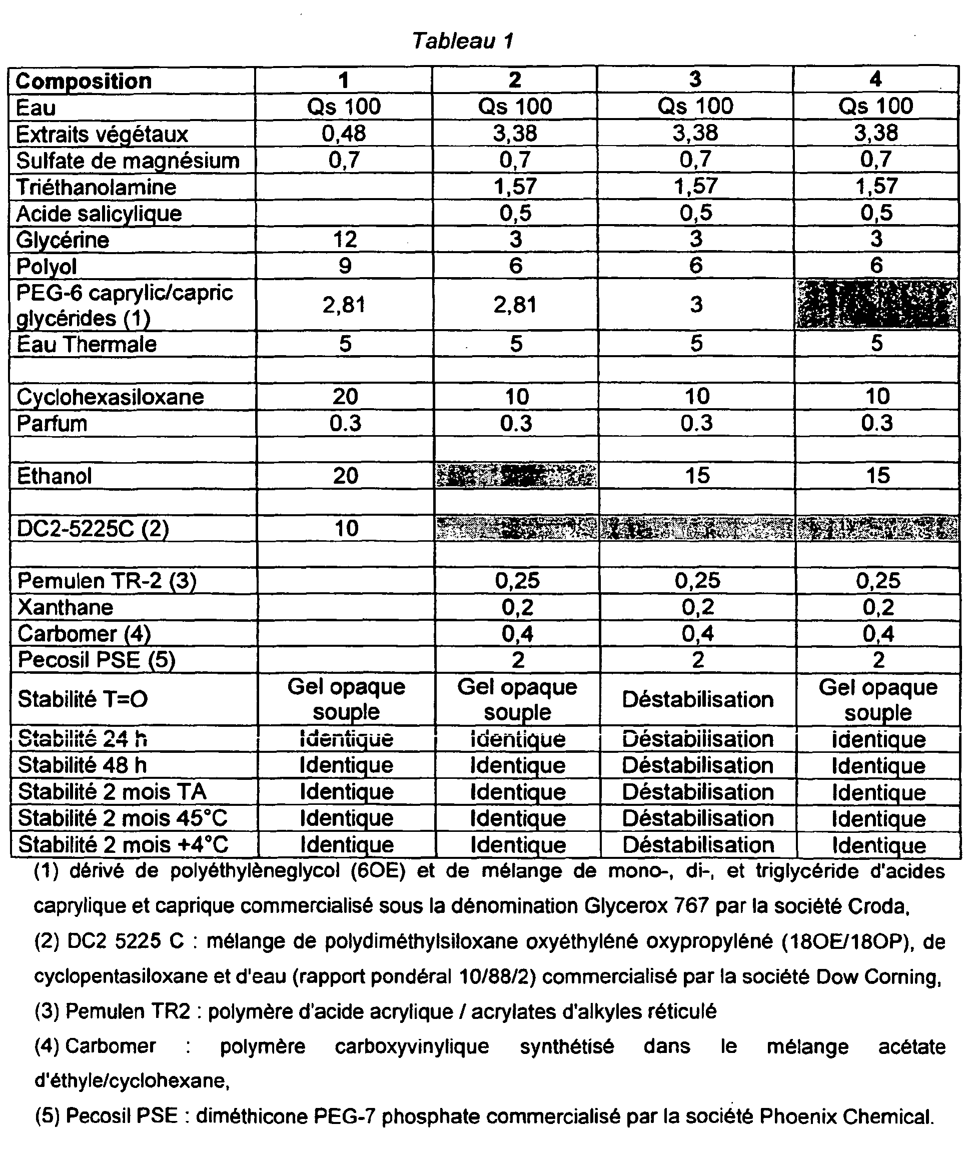

La composition dépourvue d'alcool est stable (composition 2), de même que la composition contenant l'alcool sans glycéride d'acides gras (composition 4). Lorsque l'alcool est ajouté à la composition (composition 3), l'émulsion est déstabilisée. L'addition d'un émulsionnant de formule (I) selon l'invention (DC2 5225 C dans la composition 1) permet de retrouver une émulsion dont la stabilité est comparable à celle des compositions 2 et 4.

Ces résultats montrent clairement que l'émulsionnant siliconé de formule (I) permet de stabiliser la composition contenant un glycéride d'acides gras en présence de mono-alcool. De plus l'émulsion ainsi obtenue est stable dans le temps.

### Exemple B : Comparaison de la stabilité des compositions selon l'invention en fonction de l'émulsionnnant :

Le but de cet exemple est de mettre en évidence la meilleure stabilité des compositions selon l'invention contenant l'émulsionnant siliconé de formule (I) en comparaison avec d'autres émulsionnants connus.

La composition 1 (identique à celle de la composition 1 de l'exemple A) a été réalisée de manière classique pour l'homme du métier. Elle comporte l'association de glycérides d'acides gras, d'éthanol et d'un émulsionnant de formule (I) selon la présente invention. La composition obtenue présente un bonne stabilité comme le montrent les observations directes ainsi que l'observation microscopique.

Les compositions 5 à 8, préparées de la même façon, contiennent d'autres émulsionnants eau-dans-silicone classiquement utilisés. Comme le montrent les résultats regroupés dans le tableau 2 ci-dessous, les émulsions obtenues avec ces émulsionnants ne sont pas stables et ne présentent pas un aspect acceptable à l'observation (les quantités sont exprimées en pourcentage en poids) :

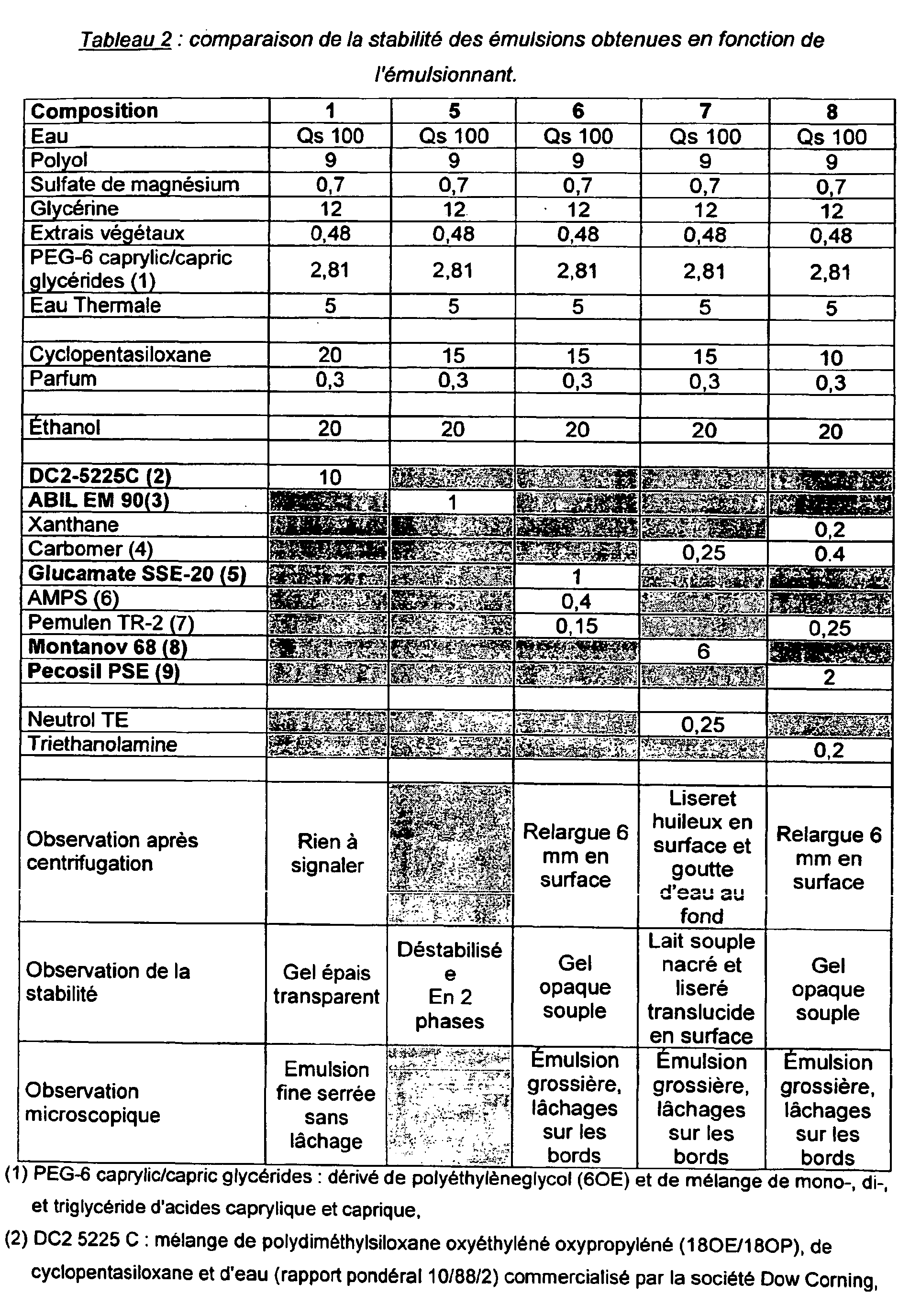

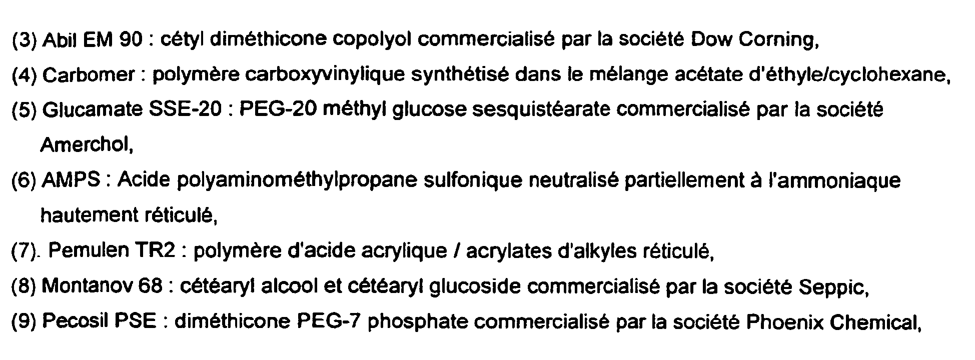

### Exemple C : Comparaison de la stabilité des compositions selon l'invention en fonction de la quantité d'alcool et de triglycérides d'acides gras :

Les compositions 9 à 11 sont préparées de manière analogue à celle des exemples précédents (les quantités sont exprimées en pourcentage en poids). La composition 9 contient 18,7 % en poids de glycérides d'acides gras. La composition 10 contient 39,45 % en poids d'éthanol, et ne contient pas d'eau. La composition 11 contient 10 % en poids de cyclopentasiloxane. La composition 1 est identique à celle des exemples A et B.

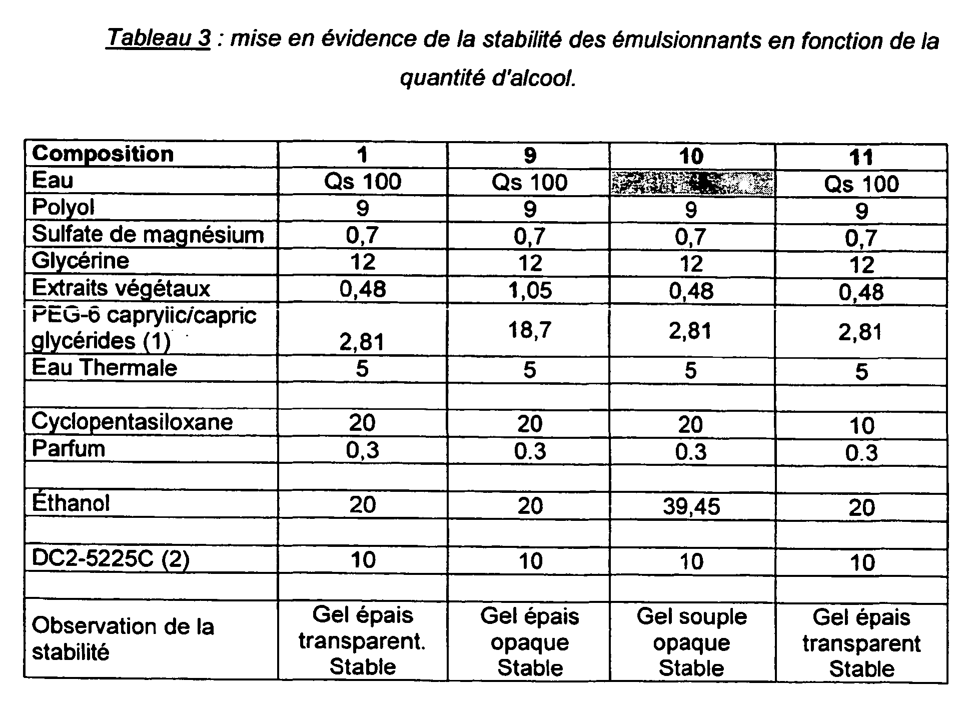

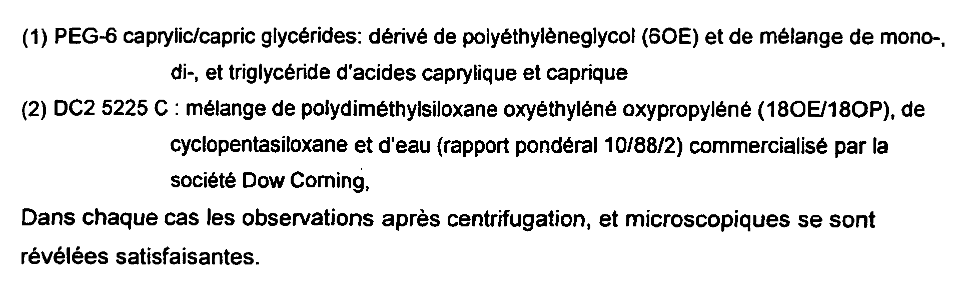

Les résultats ci-dessus montrent que l'émulsionnant siliconé de formule (I) selon la présente invention permet d'obtenir une composition stable même lorsque la quantité de glycérides d'acides gras augmente (composition 9) ou que la composition contient près de 40 % de mono-alcool (composition 10). De plus la quantité de cyclométhicone n'est pas déterminante pour la stabilité de l'émulsion (composition 11).

### Exemple D : Compositions contenant l'émulsionnant de formule (I) :

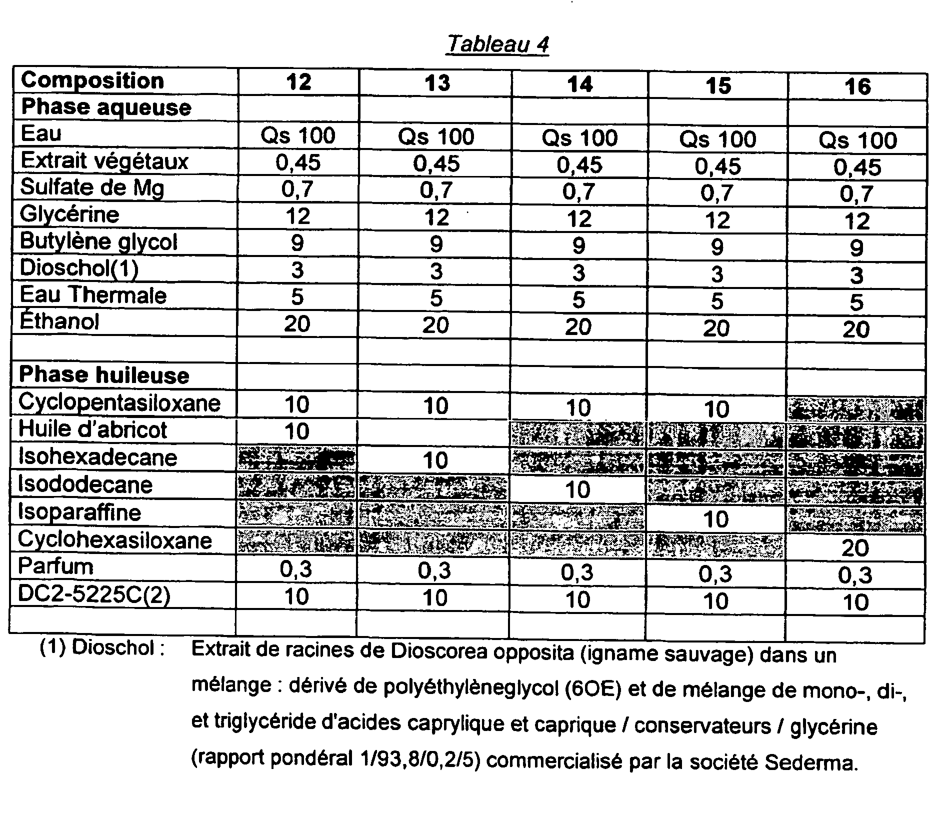

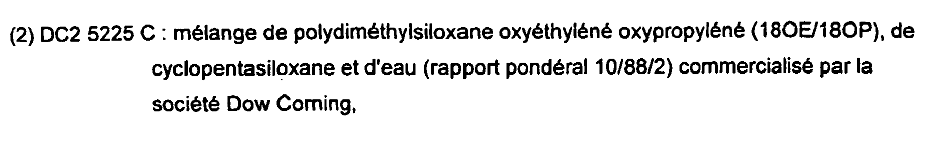

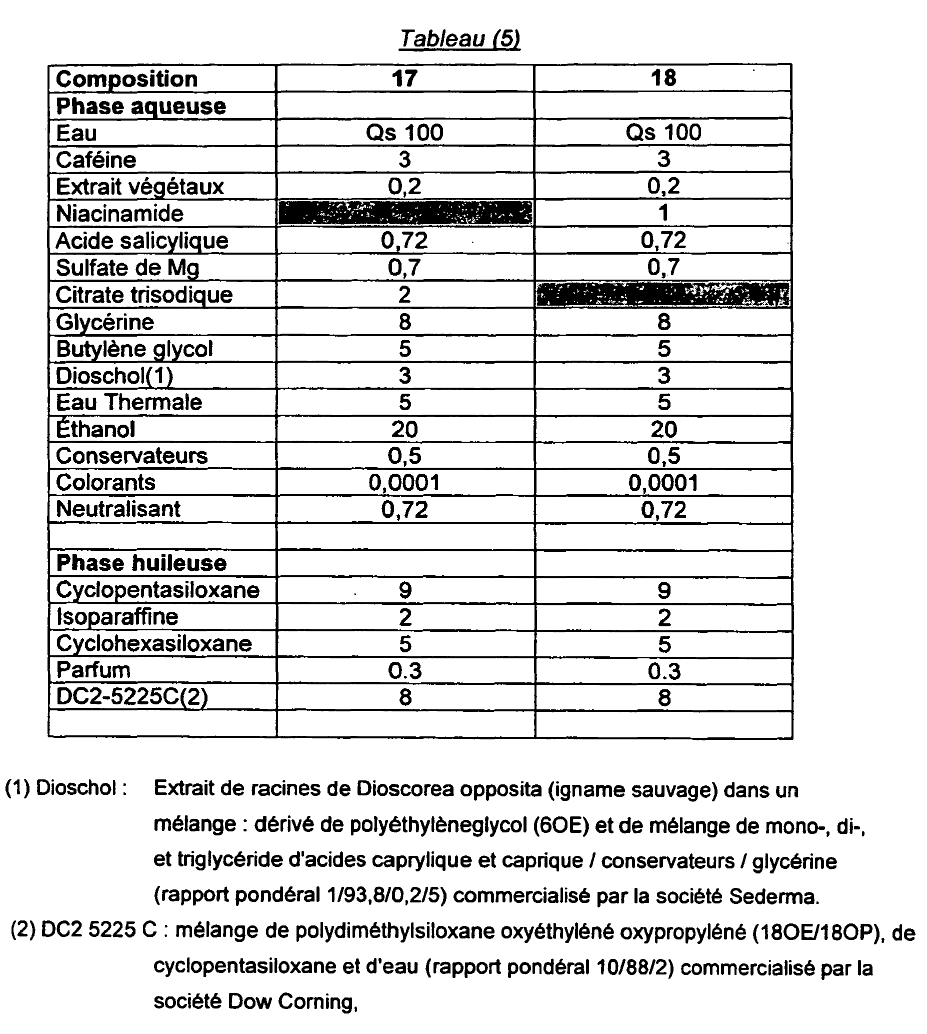

Les compositions 12 à 18 sont préparées de manière classique pour l'homme du métier :
La phase aqueuse et la phase huileuse sont préparées séparément à froid. La phase aqueuse est ensuite dispersée sous agitation vigoureuse dans la phase huileuse.

Toutes les émulsions ainsi préparées présentent des conditions de stabilité satisfaisantes à la fabrication, et dans le temps.

## Revendications

1. Composition cosmétique comprenant :
(a) au moins une phase aqueuse dispersée dans une phase grasse,
(b) au moins un glycéride d'acide gras ou de mélange d'acides gras en C₆ à C₂₂, polyoxyéthyléné et/ou polyoxypropyléné,
(c) de 10 à 50 % en poids, par rapport au poids total de la composition, d'au moins un mono-alcool en C₂-C₃,
(d) au moins un émulsionnant siliconé de formule (I) : 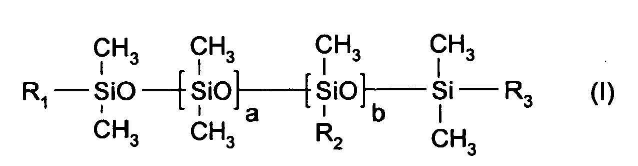 dans laquelle :
a est un nombre entier allant de 0 à 400,
b est un nombre entier allant de 0 à 50,
a et b ne pouvant être simultanément égaux à 0,
R₁, R₂, R₃, représentent indépendamment un radical alkyle en C₁-C₆, ou un radical -(CH₂)ₓ-(OCH₂CH₂)_{y}-(OCH₂CH₂CH₂)_{z}-OR₄, au moins un des radicaux R₁, R₂, R₃ étant différent d'un radical alkyle,
R₄ représente un atome d'hydrogène, un radical alkyle en C₁-C₃, ou une radical acyle en C₂-C₄,
x est un nombre entier allant de 0 à 6,
y est un nombre entier allant de 1 à 30,
z est un nombre entier allant de 0 à 30.

2. Composition selon la revendication 1, **caractérisée en ce que**, dans le composé de formule (I), R₁ et R₃ représentent chacun un radical méthyle, et R₂ un radical -(CH₂)ₓ-(OCH₂CH₂)_{y}-(OCH₂CH₂CH₂)_{z}-OR₄ dans lequel a est un entier allant de 300 à 400, b est un entier allant de 1 à 10, et y et z sont des entiers allant de 10 à 20 choisis indépendamment.

3. Composition selon la revendication 2 **caractérisée en ce que** R₄ est un atome d'hydrogène, et x un entier allant de 2 à 4.

4. Composition selon l'une des revendications 1 à 3 **caractérisée en ce que** l'émulsionnant siliconé est représenté par la formule (II) : 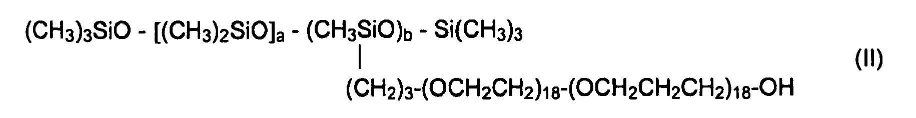 dans laquelle a est un entier allant de 300 à 400, et b est un entier allant de 1 à 10.

5. Composition selon la revendication 4 **caractérisée en ce que** dans le composé de formule (II), a vaut 394 et b vaut 4.

6. Composition selon l'une des revendications 1 à 5 **caractérisée en ce que** l'émulsionnant siliconé est présent dans la composition selon l'invention en une quantité comprise entre 0,1 % et 5% en poids par rapport au poids total de la composition, préférentiellement en une quantité comprise entre 0,5 et 3 % en poids.

7. Composition selon l'une des revendications 1 à 6 **caractérisée en ce que** l'émulsionnant siliconé de formule (I) est associé à une cyclométhicone de formule (III) : 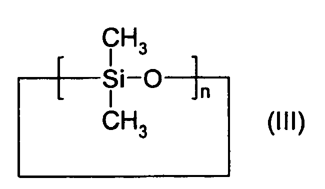 dans laquelle n est un nombre entier compris entre 3 et 8.

8. Composition selon la revendication 7 **caractérisée en ce que** la cyclométhicone est choisie parmi le cyclotétrasiloxane (n=4), le cyclopentasiloxane (n=5), et le cyclohexasiloxane (n=6).

9. Composition selon l'une des revendications 7 ou 8 **caractérisée en ce que** la cyclométhicone est présente en une quantité allant de 5 à 40 % en poids par rapport au poids total de la composition, préférentiellement de 10 à 30 % en poids.

10. Composition selon l'une des revendications 7 à 9 **caractérisée en ce que** le rapport entre l'émulsionnant siliconé de formule (I) et la cyclométhicone varie entre 0,0025 et 1, plus particulièrement entre 0,016 et 0,3.

11. Composition selon l'une des revendications 7 à 10 **caractérisée en ce que** le système émulsionnant est un mélange de composé de formule (II), de cyclopentasiloxane et d'eau dans un rapport pondéral 10/88/2.

12. Composition selon l'une des revendications 1 à 11 **caractérisée en ce que** le glycéride d'acide gras polyoxyéthyléné et/ou polyoxypropyléné est un glycéride de mélange d'acides caprylique et caprique polyoxyéthyléné et/ou polyoxypropyléné.

13. Composition selon la revendication 12 **caractérisée en ce que** le glycéride d'acide gras est un dérivé de polyéthylèneglycol (6OE) et de mélange de mono-, di-, et triglycéride d'acides caprylique et caprique.

14. Composition selon l'une des revendications 1 à 13, **caractérisée en ce que** le glycéride d'acide gras ou de mélange d'acides gras polyoxyéthyléné et/ou polyoxypropyléné est présent en une quantité comprise entre 0,001 % et 20 %, préférentiellement entre 0,1 % et 10 % en poids par rapport au poids total de la composition.

15. Composition selon l'une des revendications 1 à 14 **caractérisée en ce que** le mono-alcool est présent dans une quantité comprise entre 10 à 40 % en poids par rapport au poids total de la composition.

16. Composition selon l'une des revendications 1 à 15 **caractérisée en ce que** le mono-alcool est l'éthanol.

17. Composition selon l'une des revendications 1 à 16 **caractérisée en ce qu'**elle contient en outre, au moins un actif lipolytique ou ayant une activité favorable, directe ou indirecte, sur la diminution du tissu adipeux.

18. Composition selon la revendication 17 **caractérisée en ce que** le ou les actifs lipolytiques sont choisis parmi la caféine et ses dérivés, le citrate de caféine, la théophylline et ses dérivés, la théobromine, l'acéfylline, l'aminophylline, la chloroéthylthéophylline, le diprofylline, le diniprophylline, l'étamiphylline et ses dérivés, l'étofylline, la proxyphylline, l'éphédrine et ses dérivés, les associations de caféine et de silanol, les composés d'origine naturelle contenant des bases xanthiques tels que les extraits de thé, de café, de guarana, de maté, de cola (*Cola Nitida*); les extraits végétaux de *Garcinia Cambogia*, les extraits de *Bupleurum chinensis*, les extraits de lierre grimpant (*Hedera Helix*), d'arnica (*Arnica Montana L*), de romarin (*Rosmarinus officinalis N*), de souci *(Calendula officinalis),* de sauge (*Salvia officinalis L*), de ginseng *(Panax ginseng),* de millepertuis (*Byperycum Perforatum*), de fragon *(Ruscus aculeatus L),* d'ulmaire (*Filipendula ulmaria L*), d'orthosiphon (*Orthosiphon Stamincus Benth*), de bouleau *(Betula alba),* de cécropia et d'arganier, les extraits de ginkgo biloba, les extraits de prêle, les extraits d'escine, les complexes de phospholipide et de proanthocyanidines d'écorce de marron d'inde, les extraits de cangzhu, les extraits de *chrysanthellum indicum*, les sapogénines telles que la diosgénine ou l'hécogénine leurs dérivés et les extraits naturel en contenant, en particulier le Wild Yam, les extraits des plantes du genre *Armeniacea, Atractylodis Platicodon, Sinom-menum, Pharbitidis, Flemingia*, les extraits de *Coleus* tels que *C. Forskohlii, C. blumei, C. esquirolii, C. scutellaroïdes* , *C. xanthantus* et *C. Barbatus*, les extraits de Ballote, les extraits de *Guioa,* de *Davallia*, de *Terminalia*, de *Barringtonia*, de *Trema, d'Antirobia*, les extraits d'algues ou de phytoplancton tels que le rhodystérol ou l'extrait de *Laminaria Digitata*, l'algue skeletonema et les diatomées.

19. Composition selon la revendication 18, **caractérisée en ce que** l'actif lipolytique est un extrait de Dioscorée riche en diosgénine provenant de racines d'igname sauvage.

20. Composition selon l'une des revendications 17 à 19 **caractérisée en ce que** le ou les actifs lipolytiques sont présents en une concentration allant de 0,001 à 20 % et de préférence de 0,1 à 10 % en poids par rapport au poids total de la composition.

21. Composition selon l'une quelconque des revendication 1 à 20 **caractérisée en ce qu'**elle contient au moins un actif classiquement utilisé en cosmétique choisi parmi les agents desquamants capables d'agir, soit en favorisant l'exfoliation, soit sur les enzymes impliquées dans la desquamation ou la dégradation des cornéodesmosomes, les agents hydratants, les agents dépigmentants ou pro-pigmentants, les agents anti-glycation, les inhibiteurs de NO-synthase, les inhibiteurs de 5a-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les actifs lipolytiques ou ayant une activité favorable, directe ou indirecte, sur la diminution du tissu adipeux, les agents agissant sur la microcirculation, et les agents agissant sur le métabolisme énergétique des cellules.

22. Procédé non-thérapeutique de traitement cosmétique de la peau, des cheveux, des ongles, du cuir chevelu et/ou des muqueuses, **caractérisé par le fait que** l'on applique sur la peau, les cheveux, les ongles, le cuir chevelu et/ou les muqueuses, une composition selon l'une des revendications 1 à 21.

## Claims

1. Cosmetic composition comprising:
(a) at least one aqueous phase dispersed in a fatty phase,
(b) at least one glyceride of a C₆ to C₂₂ fatty acid or of a mixture of C₆ to C₂₂ fatty acids, which is polyoxyethylenated and/or polyoxypropylenated,
(c) from 10% to 50% by weight, relative to the total weight of the composition, of at least one C₂-C₃ monoalcohol,
(d) at least one silicone emulsifier of formula (I) : 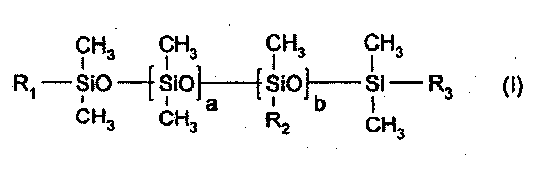 in which:
a is an integer ranging from 0 to 400,
b is an integer ranging from 0 to 50,
a and b cannot simultaneously be equal to 0,
R₁, R₂ and R₃ independently represent a C₁-C₆ alkyl radical or a radical - (CH₂)ₓ-(OCH₂CH₂)_{y}-(OCH₂CH₂CH₂)_{z}-OR₄, at least one of the radicals R₁, R₂ and R₃ being other than an alkyl radical,
R₄ represents a hydrogen atom, a C₁-C₃ alkyl radical or a C₂-C₄ acyl radical,
x is an integer ranging from 0 to 6,
y is an integer ranging from 1 to 30,
z is an integer ranging from 0 to 30.

2. Composition according to Claim 1, **characterized in that,** in the compound of formula (I), R₁ and R₃ each represent a methyl radical, and R₂ represents a radical -(CH₂)ₓ-(OCH₂CH₂)_{y}-(OCH₂CH₂CH₂)_{z}-OR₄ in which a is an integer ranging from 300 to 400, b is an integer ranging from 1 to 10, and y and z are integers ranging from 10 to 20, chosen independently.

3. Composition according to Claim 2, **characterized in that** R₄ is a hydrogen atom and x is an integer ranging from 2 to 4.

4. Composition according to one of Claims 1 to 3, **characterized in that** the silicone emulsifier is represented by formula (II): 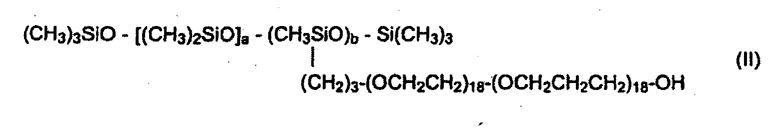 in which a is an integer ranging from 300 to 400 and b is an integer ranging from 1 to 10.

5. Composition according to Claim 4, **characterized in that**, in the compound of formula (II), a is 394 and b is 4.

6. Composition according to one of Claims 1 to 5, **characterized in that** the silicone emulsifier is present in the composition according to the invention in an amount of between 0.1% and 5% by weight and preferably in an amount of between 0.5% and 3% by weight relative to the total weight of the composition.

7. Composition according to one of Claims 1 to 6, **characterized in that** the silicone emulsifier of formula (I) is combined with a cyclomethicone of formula (III): 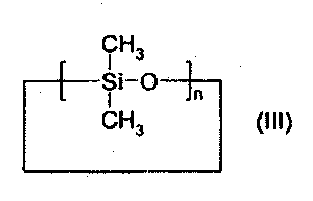 in which n is an integer between 3 and 8.

8. Composition according to Claim 8, **characterized in that** the cyclomethicone is chosen from cyclotetrasiloxane (n = 4), cyclopentasiloxane (n = 5) and cyclohexasiloxane (n = 6).

9. Composition according to either of Claims 7 and 8, **characterized in that** the cyclomethicone is present in an amount ranging from 5% to 40% by weight and preferably from 10% to 30% by weight relative to the total weight of the composition.

10. Composition according to one of Claims 7 to 9, **characterized in that** the ratio between the silicone emulsifier of formula (I) and the cyclomethicone ranges between 0.0025 and 1 and more particularly between 0.016 and 0.3.

11. Composition according to one of Claims 7 to 10, **characterized in that** the emulsifying system is a mixture of a compound of formula (II), cyclopentasiloxane and water in a 10/88/2 weight ratio.

12. Composition according to one of Claims 1 to 11, **characterized in that** the polyoxyethylenated and/or polyoxypropylenated fatty acid glyceride is a glyceride of a mixture of caprylic and capric acids, which is polyoxyethylenated and/or polyoxypropylenated.

13. Composition according to Claim 12, **characterized in that** the fatty acid glyceride is a derivative of polyethylene glycol (6EO) and of a mixture of caprylic and capric acid mono-, di- and triglyceride.

14. Composition according to one of Claims 1 to 13, **characterized in that** the glyceride of a fatty acid or of a mixture of fatty acids, which is polyoxyethylenated and/or polyoxypropylenated, is present in an amount of between 0.001% and 20% and preferably between 0.1% and 10% by weight relative to the total weight of the composition.

15. Composition according to one of Claims 1 to 14, **characterized in that** the monoalcohol is present in an amount of between 10% and 40% by weight relative to the total weight of the composition.

16. Composition according to one of Claims 1 to 15, **characterized in that** the monoalcohol is ethanol.

17. Composition according to one of Claims 1 to 16, **characterized in that** it also contains at least one lipolytic active agent or an agent that has a direct or indirect favourable activity on decreasing adipose tissue.

18. Composition according to Claim 17, **characterized in that** the lipolytic active agent(s) is (are) chosen from caffeine and its derivatives, caffeine citrate, theophylline and its derivatives, theobromine, acefylline, aminophylline, chloroethyltheophylline, diprofylline, diniprophylline, etamiphylline and its derivatives, etofylline, proxyphylline, ephedrine and its derivatives, combinations of caffeine and of silanol, compounds of natural origin containing xanthine bases, such as extracts of tea, of coffee, of guarana, of maté, of cola (*Cola nitida*); plant extracts of *Garcinia cambogia*, extracts of *Bupleurum chinensis*, extracts of climbing ivy (*Hedera helix*), of arnica (*Arnica montana L*), of rosemary (*Rosmarinus officinalis N*), of marigold (*Calendula officinalis*), of sage (*Salvia officinalis L*), of ginseng (*Panax ginseng*), of St. John's wort (*Hypericum perforatum*), of butcher's broom *(Ruscus aculeatus L),* of meadowsweet (*Filipendula ulmaria L*), of orthosiphon (*Orthosiphon stamincus benth*), of birch (*Betula alba*), of pumpwood and of argan tree, extracts of ginkgo biloba, extracts of horsetail, extracts of escin, complexes of phospholipid and of proanthocyanidins from horse chestnut bark, extracts of changzhou, extracts of *Chrysanthellum indicum*, sapogenins such as diosgenin or hecogenin, derivatives thereof and natural extracts containing them, in particular wild yam, extracts of plants of the genus *Armeniacea, Atractylodis platicodon, Sinom-menum, Pharbitidis* or *Flemingia*, extracts of *Coleus* such as *C. forskohlii, C. blumei, C. esquirolii, C. scutellaroides, C. xanthantus* and *C. barbatus*, extracts of Ballota, extracts of *Guioa,* of *Davallia*, of *Terminalia*, of *Barringtonia*, of *Trema*, or of *Antirobia*, extracts of algae or of phytoplankton, such as rhodysterol or the extract of *Laminaria digitata*, the skeletonema alga and diatomaceous earths.

19. Composition according to Claim 18, **characterized in that** the lipolytic active agent is a diosgenin-rich Dioscorea extract originating from wild yam root.

20. Composition according to one of Claims 17 to 19, **characterized in that** the lipolytic active agent(s) is (are) present in a concentration ranging from 0.001% to 20% and preferably from 0.1% to 10% by weight relative to the total weight of the composition.

21. Composition according to any one of Claims 1 to 20, **characterized in that** it contains at least one active agent conventionally used in cosmetics, chosen from desquamating agents capable of acting either by promoting exfoliation, or on the enzymes involved in desquamation or the degradation of corneodesmosomes, moisturizers, depigmenting or propigmenting agents, anti-glycation agents, NO-synthase inhibitors, 5α-reductase inhibitors, lysyl and/or prolyl hydroxylase inhibitors, agents for stimulating the synthesis of dermal or epidermal macromolecules and/or for preventing their degradation, agents for stimulating the proliferation of fibroblasts or keratinocytes and/or the differentiation of keratinocytes, muscle relaxants, antimicrobial agents, tensioning agents, antipollution agents or free-radical scavengers, antiinflammatories, lipolytic active agents or agents that have a direct or indirect favourable activity on decreasing adipose tissue, agents acting on the capillary circulation, and agents acting on the energy metabolism of cells.

22. Nontherapeutic process for cosmetic treatment of the skin, the hair, the nails, the scalp and/or the mucous membranes, **characterized in that** a composition according to one of Claims 1 to 21 is applied to the skin, the hair, the nails, the scalp and/or the mucous membranes.

## Patentansprüche

1. Kosmetische Zusammensetzung, die enthält:
(a) mindestens eine wässrige Phase, die in einer Fettphase dispergiert ist,
(b) mindestens ein polyethoxyliertes und/oder polypropoxyliertes Glycerid einer Fettsäure oder eines Gemisches von Fettsäuren mit 6 bis 22 Kohlenstoffatomen,
(c) 10 bis 50 Gew.-% mindestens eines Monoalkohols mit 2 bis 3 Kohlenstoffatomen, bezogen auf das Gesamtgewicht der Zusammensetzung,
(d) mindestens einen Siliconemulgator der Formel (I): 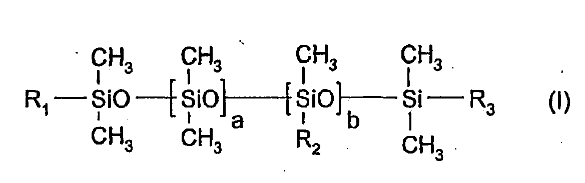 in der Formel:
a bedeutet 0 oder eine ganze Zahl von 1 bis 400,
b bedeutet 0 oder eine ganze Zahl von 1 bis 50,
a und b können nicht gleichzeitig 0 sein,
R₁, R₂, R₃ bedeuten unabhängig voneinander eine C₁₋₆-Alkylgruppe oder eine Gruppe -(CH₂)ₓ-(OCH₂CH₂)_{y}-(OCH₂CH₂CH₂)_{z}-OR₄, wobei mindestens eine der Gruppen R₁, R₂, R₃ von einer Alkylgruppe verschieden ist,
R₄ bedeutet ein Wasserstoffatom, eine C₁₋₃-Alkylgruppe oder eine C₂₋₄-Acylgruppe,
x bedeutet 0 oder eine ganze Zahl von 1 bis 6,
y ist eine ganze Zahl von 1 bis 30,
z bedeutet 0 oder eine ganze Zahl von 1 bis 30.

2. Zusammensetzung nach Anspruch 1, **dadurch gekennzeichnet, dass** in der Verbindung der Formel (I) R₁ und R₃ jeweils eine Methylgruppe bedeuten und R₂ eine Gruppe -(CH₂)ₓ-(OCH₂CH₂)_{y}-(OCH₂CH₂CH₂)_{z}-OR₄ ist, wobei a eine ganze Zahl von 300 bis 400 bedeutet, b eine ganze Zahl von 1 bis 10 ist und y und z unabhängig voneinander ganze Zahlen im Bereich von 10 bis 20 bedeuten.

3. Zusammensetzung nach Anspruch 2, **dadurch gekennzeichnet, dass** R₄ ein Wasserstoffatom ist und x eine ganze Zahl von 2 bis 4 bedeutet.

4. Zusammensetzung nach einem der Ansprüche 1 bis 3, **dadurch gekennzeichnet, dass** der Siliconemulgator durch die Formel (II) dargestellt wird: 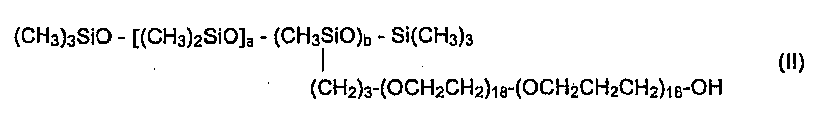 worin a eine ganze Zahl von 300 bis 400 bedeutet und b eine ganze Zahl von 1 bis 10 ist.

5. Zusammensetzung nach Anspruch 4, **dadurch gekennzeichnet, dass** in der Verbindung der Formel (II) a 394 bedeutet und b 4 ist.

6. Zusammensetzung nach einem der Ansprüche 1 bis 5, **dadurch gekennzeichnet, dass** der Siliconemulgator in der erfindungsgemäßen Zusammensetzung in einem Mengenanteil im Bereich von 0,1 bis 5 Gew.-%, bezogen auf das Gesamtgewicht der Zusammensetzung, und vorzugsweise in einem Mengenanteil von 0,5 bis 3 Gew.-% enthalten ist.

7. Zusammensetzung nach einem der Ansprüche 1 bis 6, **dadurch gekennzeichnet, dass** der Siliconemulgator der Formel (I) mit einem Cyclomethicon der Formel (III) kombiniert wird: 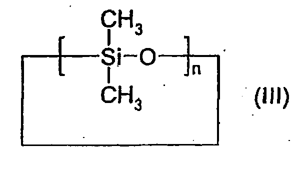 worin n eine ganze Zahl von 3 bis 8 bedeutet.

8. Zusammensetzung nach Anspruch 7, **dadurch gekennzeichnet, dass** das Cyclomethicon unter Cyclotetrasiloxan (n=4), Cyclopentasiloxan (n=5) und Cyclohexasiloxan (n=6) ausgewählt ist.

9. Zusammensetzung nach einem der Ansprüche 7 oder 8, **dadurch gekennzeichnet, dass** das Cyclomethicon in einem Mengenanteil von 5 bis 40 Gew.-%, bezogen auf das Gesamtgewicht der Zusammensetzung, und vorzugsweise 10 bis 30 Gew.-% vorliegt.

10. Zusammensetzung nach einem der Ansprüche 7 bis 9, **dadurch gekennzeichnet, dass** das Verhältnis des Siliconemulgators der Formel (I) und des Cyclomethicons im Bereich von 0,0025 bis 1 und insbesondere 0,016 bis 0,3 liegt.

11. Zusammensetzung nach einem der Ansprüche 7 bis 10, **dadurch gekennzeichnet, dass** das Emulgatorsystem ein Gemisch aus der Verbindung der Formel (II), Cyclopentasiloxan und Wasser in einem Gewichtsverhältnis 10/88/2 ist.

12. Zusammensetzung nach einem der Ansprüche 1 bis 11, **dadurch gekennzeichnet, dass** das polyethoxylierte und/oder polypropoxylierte Fettsäureglycerid ein polyethoxyliertes und/oder polypropoxyliertes Glycerid des Gemisches von Caprylsäure und Caprinsäure ist.

13. Zusammensetzung nach Anspruch 12, **dadurch gekennzeichnet, dass** das Fettsäureglycerid ein Derivat von Polyethylenglycol (6 EO) und des Gemisches des Mono-, Di- und Triglycerids von Caprylsäure und Caprinsäure ist.

14. Zusammensetzung nach einem der Ansprüche 1 bis 13, **dadurch gekennzeichnet, dass** das Glycerid einer Fettsäure oder eines Gemisches von Fettsäuren, das polyethoxyliert und/oder polypropoxyliert ist, in einem Mengenanteil von 0,001 bis 20 Gew.-% und vorzugsweise 0,1 bis 10 Gew.-%, bezogen auf das Gesamtgewicht der Zusammensetzung, vorliegt.

15. Zusammensetzung nach einem der Ansprüche 1 bis 14, **dadurch gekennzeichnet, dass** der Monoalkohol in einem Mengenanteil von 10 bis 40 Gew.-%, bezogen auf das Gesamtgewicht der Zusammensetzung, enthalten ist.

16. Zusammensetzung nach einem der Ansprüche 1 bis 15, **dadurch gekennzeichnet, dass** es sich bei dem Monoalkohol um Ethanol handelt.

17. Zusammensetzung nach einem der Ansprüche 1 bis 16, **dadurch gekennzeichnet, dass** sie ferner mindestens einen lipolytischen Wirkstoff oder einen Wirkstoff enthält, der direkt oder indirekt eine günstige Wirkung auf die Verminderung des Fettgewebes hat.

18. Zusammensetzung nach Anspruch 17, **dadurch gekennzeichnet, dass** der oder die lipolytischen Wirkstoffe unter Coffein und seinen Derivaten, Coffeincitrat, Theophyllin und seinen Derivaten, Theobromin, Acefyllin, Aminophyllin, Chlorethyltheophyllin, Diprofyllin, Diniprophyllin, Etamiphyllin und seinen Derivaten, Etofyllin, Proxyphyllin, Ephedrin und seinen Derivaten, Kombinationen von Coffein und Silanol, Verbindungen natürlicher Herkunft, die Xanthinbasen enthalten, wie die Extrakte von Tee, Kaffee, Guarana, Mate, Cola (*Cola nitida*); Pflanzenextrakten von *Garcinia cambogia*, Extrakten von *Bupleurum chinensis*, Extrakten von Efeu (*Hedera helix*), Arnika (*Arnica montana L*), Rosmarin (*Rosmarinus officinalis N*), Ringelblume *(Calendula officinalis*), Salbei (*Salvia officinalis L*), Ginseng *(Panax ginseng),* Johanniskraut (*Hypericum perforatum*), Mäusedorn (*Ruscus aculeatus L),* Mädesüß (*Filipendula ulmaria L*), Orthosiphon (*Orthosiphon stamincus Benth*), Birke (*Betula alba*), Cecropia und Arganbaum, Extrakten von *Ginkgo biloba,* Schachtelhalmextrakten, Aescinextrakten, Komplexen von Phospholipid und Proanthocyanidinen der Rosskastanienrinde, Extrakten von Cangzhu, Extrakten von *Chrysanthellum indicum*, Sapogeninen wie Diosgenin oder Hecogenin, deren Derivaten und natürlichen Extrakten, die sie enthalten, besonders Wild Yam, Extrakten von Pflanzen der Gattung *Armeniacea, Atractylodis platicondon, Sinom-menum, Pharbitidis, Flemingia*, Extrakten von *Coleus,* wie *C. forskohlii, C. blumei, C. esquirolii, C. scutellaroides, C. xanthantus* und *C. Barbatus*, Extrakten von Ballote, Extrakten von *Guioa,* Extrakten von *Davallia, Terminalia, Barringtonia, Trema, Antirobia*, Extrakten von Algen oder Phytoplankton, wie Rhodysterol oder dem Extrakt von *Laminaria digitata*, der Alge Skeletonema und Kieselalgen, ausgewählt sind.

19. Zusammensetzung nach Anspruch 18, **dadurch gekennzeichnet, dass** der lipolytische Wirkstoff ein Diosgenin-reicher Dioscorea-Extrakt ist, der aus den Wurzeln von Wildem Yams stammt.

20. Zusammensetzung nach einem der Ansprüche 17 bis 19, **dadurch gekennzeichnet, dass** der oder die lipolytischen Wirkstoffe in einer Konzentration von 0,001 bis 20 Gew.-% und vorzugsweise 0,1 bis 10 Gew.-%, bezogen auf das Gesamtgewicht der Zusammensetzung, enthalten sind.

21. Zusammensetzung nach einem der Ansprüche 1 bis 20, **dadurch gekennzeichnet, dass** sie mindestens einen Wirkstoff enthält, der herkömmlich in der Kosmetik verwendet wird und ausgewählt ist unter den abschuppenden Wirkstoffen, die befähigt sind, entweder die Exfoliation zu fördern oder auf die bei der Abschuppung oder Zersetzung der Corneodesmosomen beteiligten Enzyme einzuwirken, Hydratisierungsmitteln, depigmentierenden oder pigmentierungsfördernden Wirkstoffen, Antiglycationswirkstoffen, Inhibitoren der NO-Synthase, Inhibitoren der 5α-Reduktase, Inhibitoren der Lysyl- und/oder Prolylhydroxylase, Wirkstoffen, die die Synthese von Makromolekülen der Dermis oder Epidermis stimulieren und/oder ihre Zersetzung verhindern, Wirkstoffen, die die Proliferation von Fibroblasten oder Keratinocyten und/oder die Differenzierung von Keratinocyten stimulieren, Muskelrelaxantien, antimikrobiellen Wirkstoffen, straffenden Wirkstoffen, Antipollutionswirkstoffen oder Radikalfängern für freie Radikale, entzündungshemmenden Wirkstoffen, lipolytischen Wirkstoffen oder Wirkstoffen, die direkt oder indirekt eine günstige Wirkung bezüglich der Verminderung des Fettgewebes haben, Wirkstoffen, die auf die Mikrozirkulation einwirken, und Wirkstoffen, die auf den Energiehaushalt der Zellen einwirken.

22. Nichttherapeutisches kosmetisches Verfahren zur Behandlung der Haut, der Haare, der Nägel, der Kopfhaut und/oder der Schleimhäute, **dadurch gekennzeichnet, dass** auf die Haut, die Haare, die Nägel, die Kopfhaut und/oder die Schleimhäute eine Zusammensetzung nach einem der Ansprüche 1 bis 21 aufgetragen wird.
